# EUROPEAN PATENT SPECIFICATION

(11) **EP 4 424 287 B1**
(45) Date of publication and mention of the grant of the patent: **02.12.2026**
(21) Application number: 24180633.0
(22) Date of filing: 21.12.2018
(51) Int. Cl.: A61F 13/02

(54) **SUPERABSORBENT WOUND DRESSING WITH SILICONE WOUND CONTACT LAYER**
SUPERABSORBIERENDER WUNDVERBAND MIT SILIKONWUNDKONTAKTSCHICHT
PANSEMENT SUPERABSORBANT AVEC COUCHE DE CONTACT EN SILICONE

(43) Date of publication of application: 04.09.2024
(62) Document number(s) of the earlier application(s) in accordance with Art. 76 EPC: 18215477.3 / 3 669 844
(73) Proprietor: Paul Hartmann AG, 89522 Heidenheim (DE)
(72) Inventor: MERCKEL, Fabien, 67100 Straßbourg (FR); THIEBAUT, Renaud, 67730 Chatenois (FR)
(74) Representative: Paul Hartmann AG Patents & Licensing

(56) References cited:
- EP-A2- 2 324 803
- WO-A1-2017/196888
- US-A- 5 356 678
- US-A1- 2014 114 268
- US-A1- 2016 067 107

## Description

The present invention deals with an absorbent wound dressing, in particular for the treatment of chronic wounds.

Absorbent wound dressings have been used in the treatment of wounds for several years. Ideal wound dressings must fulfill multiple tasks that sometimes are difficult or impossible to optimize simultaneously. They must be capable to absorb high amounts of wound exudate in a rapid and complete manner, providing a wound-healing supporting environment, while preventing leakage of wound exudate during treatment. They must be able to adhere to intact human or animal skin without irritating the wound tissue or the intact skin surrounding the wound and must exhibit atraumatic properties, i.e. they must be displaced from the wound without causing pain or irritation. They must provide a barrier against external dust, moisture and microorganisms, but have to be moisture-permeable in order to enable sufficient gas and vapor exchange between the wound and the environment. The dressings must exhibit comfortable wearing properties, i.e. they must be flexible and capable of adapting to different skin regions of the body thereby not causing pain or unpleasant feeling during the treatment. Furthermore, they should be able to be worn for up to seven days without dressing change.

Currently, no wound dressing is available on the market which exhibits each of these properties in a perfect matter.

WO93/19709 discloses a method and an arrangement for manufacturing wound dressings, wherein the wound dressings comprise a perforated wound contact layer made from an adhesive silicone.

WO93/19710 discloses a wound dressing comprising a backing layer, an absorbent pad and a wound contact layer, wherein the wound contact layer comprises a perforated layer of adhesive silicone.

WO2014/097069 discloses a wound dressing comprising a backing layer, an absorbent island and an apertured wound contact layer.

US2016/0067107 discloses an absorbent wound dressing comprising a superabsorbent material that is in fluid communication with the surface of a wound to which the dressing is applied when in use wherein the wound-facing surface is from a yarn comprising gelling fibres.

US2014/0114268 discloses an absorbent dressing comprising an adhesive silicone-coated side strip wherein the dressing includes an absorbent non-woven fabric and a breathable impermeable substrate which is formed by assembling a continuous film and an openwork reinforcement that is coated with an adhesive silicone gel.

EP2324803 A2 discloses an item comprising a patch of releasably adhesive laminate comprising a structural layer carrying on at least part of one side thereof a hydrophobic gel and on at least part of the other side thereof a pressure-sensitive adhesive.

WO2017196888 discloses a dressing which includes an adhesive layer for adhering the dressing adjacent the wound, a wound contact layer, a pressure dispersion layer, a plurality of layers of absorbent material disposed between the wound contact layer and the pressure dispersion layer, a backing layer and a flexible connector disposed on the second surface of the backing layer.

US5356678A discloses a pouch for absorbing liquids which contains an inner layer of water-insoluble, liquid-absorbing material, such as cross-linked sodium polyacrylate wherein the pouch is formed by application of heat sealing.

There is a strong need for improvements of one or more of the mentioned properties in the development of wound dressings, in particular in the treatment of chronic wounds.

The invention according to claim 1 solves the aforementioned problems.

The wound dressing comprises a backing layer, an absorbent pad and a wound contact layer.

In the present application the terms proximal side or proximal surface are to be understood as that side or surface of a layer or material of the wound dressing that is in use facing a wound. The terms distal side or distal surface are to be understood as that side or surface of a layer or material that is in use facing away from the wound.

The backing layer may have any shape, such as square, rectangular, circular, oval, trapezium-shaped, suitably with rounded corners.

The backing layer supports the absorbent pad and provides a barrier to passage of microorganisms through the dressing. The backing layer is substantially liquid-impermeable but permeable for water vapour, suitably in form of a film material. The backing layer suitably has a moisture vapor transmission rate (MVTR) from 300 to 30000 g/m²/24h, suitably 1000 to 15000 g/m²/24h, and in one embodiment 1000 to 5000 g/m²/24h according to test method EN 13726. The backing layer has a thickness of 10 µm to 100 µm, suitably 12 µm to 75 µm, and in a preferred embodiment 15 µm to 50 µm, most preferred 25 µm to 35 µm. The backing layer comprises a proximal surface and a distal surface. The distal surface of the backing layer suitably is a low-friction surface in order to exhibit advantageous wearing properties without sticking to clothes or causing noise during use.

Suitable polymers for forming the backing layer material include polyurethanes, poly alkoxyalkyl acrylates and methyl acrylates such as those disclosed in GB1280631. Suitably, the backing layer comprises a continuous layer of a high density blocked polyurethane foam that is predominantly closed-cell. A suitable backing layer material is the polyurethane film available as film number 1305 from the company Coveris, having a thickness of 30 µm.

Preferably, the backing layer is transparent in order to allow a view of the absorbent pad positioned on the proximal side of the backing layer.

The backing layer can be coated with an adhesive on the proximal surface. The adhesive secures the backing layer to further layers of the wound dressing, particularly to the absorbent pad and to the wound contact layer. The backing layer can be coated with adhesive continuously, i.e. the adhesive covers the entire proximal surface of the backing layer. In different embodiments, the backing layer can be coated partially with adhesive. In these embodiments the adhesive can be in the form of stripes, dots or in different patterns. In a further embodiment the backing layer comprises an adhesive layer on the proximal surface that leaves an adhesive-free part in the center of the backing layer to reduce contact with the distal surface of the absorbent pad. The MVTR in an adhesive-free region of the backing layer is increased. If the absorbent core within the absorbent pad swells after absorption of wound exudate the absorbent pad changes its spatial extension. This can either cause delamination due to the occurrence of shearing forces within the wound dressing, or it can cause stress and pain on the wound and/or the wound surrounding skin due to shearing forces acting on the wound and skin of the patient. A dressing leaving an adhesive-free area between the distal surface of the absorbent pad and the proximal surface of the backing layer can be advantageous in this respect.

The adhesive preferably is a pressure-sensitive adhesive of the type that is conventionally used for medical dressings. Suitable pressure-sensitive adhesives can be based on acrylic acid, acrylate ester copolymers, polyvinyl ethyl ether and polyurethane such as those described in GB1280631. The basis weight of the pressure-sensitive adhesive is suitably 10 g/ m² to 100 g/ m², preferably 15 g/ m² to 50 g/m², more preferably 20 g/ m² to 30 g/m².

The absorbent pad comprises an absorbent core and an envelope surrounding the absorbent core.

The absorbent core has a proximal side and a distal side. The absorbent core comprises an absorbent material. An absorbent is capable of absorbing liquids such as wound exudate. The material can comprise any wound exudate-absorbing material, particularly absorbent fibers and/or absorbent particles.

Suitably, the material comprises a mixture of absorbent fibers and absorbent particles.

Suitable materials for absorbent fibers can be cellulose or cellulose-based polymers, viscose, polyester, polyamide, derivatives, copolymers and mixtures thereof. Furthermore, suitable fibers include superabsorbent fibers that can be based on polyacrylic acid, sodium polyacrylate, polyacrylic acid esters, copolymers and mixtures thereof. A preferred material is cellulose.

Suitable materials for absorbent particles include, but are not limited to, superabsorbent particles. Suitable materials for superabsorbent particles include, but are not limited to, polyacrylic acid, sodium polyacrylate, polyacrylic acid esters, copolymers and mixtures thereof. A preferred material is sodium polyacrylate.

In a preferred embodiment, the absorbent core comprises a mixture of absorbent fibers consisting essentially of cellulose and superabsorbent particles made from polyacrylic acid and sodium polyacrylate. Such an absorbent core exhibits rapid absorption of wound exudate and rapid distribution within the absorbent core.

The superabsorbent material can be present in an amount of from 1 % by weight to 99 % by weight within the absorbent core. Preferably, the amount of superabsorbent material comprises from 30 % to 55 % by weight of the absorbent core.

A superabsorbent material is generally understood to be a water-insoluble, swellable polymer that can absorb a multiple of its own weight of liquid such as water, saline solutions, or body fluids. The absorption of liquid results in the formation of a hydrogel. The absorption capacity for pure water is typically greater than the absorption capacity for saline liquid. In connection with this invention, the term superabsorbent material refers, in particular, to a material exhibiting a w value (free swell capacity) according to the standard test method WSP 240.2 (05) of at least 10 g/g, preferably at least 20 g/g. The test method WSP 240.2 (05) for determining the w value is described in "Standard Test Methods for the Nonwovens and Related Industries," 2008 edition (published by "EDANA, International Association Serving the Nonwovens and Related Industries," Cary, N.C., U.S.A. and "INDA, Association of the Nonwovens Fabrics Industry," Brussels, Belgium).

The absorbent pad further can comprise a diffusion layer. This layer enables an easier distribution of wound exudate in a horizontal manner within the absorbent core. This layer can be present on the proximal side of the absorbent core between the proximal layer of the envelope and the proximal surface of the absorbent core. The diffusion layer can also be present as a layer of material that envelopes the entire absorbent core. Preferably, the diffusion layer is a tissue surrounding the layer of absorbent material. In a preferred embodiment, this tissue is made from cellulose and has a weight of 15 to 20 g/m².

The envelope surrounding the absorbent core comprises a first layer that covers the proximal side of the absorbent core facing the wound in use, and a second layer that covers the distal side of the absorbent core facing away from the wound in use. The first layer of the envelope extends over the proximal side of the absorbent core and the second layer of the envelope extends over the distal side of the absorbent core, each of them forming a border area surrounding the absorbent core. The first and the second layer of the envelope of the absorbent pad are joined to each other along the border area surrounding the absorbent core.

The envelope comprises a first, proximal layer of a first liquid-permeable material and a second, distal layer.

The envelope comprises a first layer of a liquid-permeable material exhibiting hydrophilic properties. The material comprises a material originally exhibiting hydrophobic properties that is treated by a chemical or physical process to exhibit hydrophilic properties. Suitable processes for the hydrophilization of originally non-hydrophilic materials include electrochemical processes, flame treatment, corona treatment and plasma treatment. Suitable materials can be cellulose, polyester, polyamide, polyethylene, polypropylene, or copolymers from two or more of the aforementioned materials. Preferably, the first layer of hydrophilic material comprises either a polypropylene nonwoven that has been treated physically in order to exhibit hydrophilic properties or it comprises a nonwoven comprising a mixture of viscose fibers and polyamide fibers. Preferred materials can be a nonwoven comprising 55 % polyamide and 45 % viscose fibers having a weight of 37 g/ m² purchased under the name M1526 from the company Freudenberg (Germany) or a nonwoven comprising 63 % polypropylene and 37% viscose fibers having a weight of 45 g/ m² purchased under the name 670532/2 from the company Freudenberg (Germany).

In a preferred embodiment, the envelope comprises a second layer of a material exhibiting hydrophobic properties. The material can exhibit hydrophobic properties by its chemical nature or can comprise material originally exhibiting hydrophilic properties that is provided with hydrophobic properties by chemical or physical treatment, e.g. by the treatment with fluorocarbons, silicones, alkanes etc.

In a further preferred embodiment, the second layer of the envelope comprises a substantially liquid-impermeable material.

Both layers of the envelope surrounding the absorbent core can be colored in any way that is suitable. Usually, medical products have a white color signaling cleanness and purity. In one embodiment, one or both layers have a slightly green color. This color provides a strong color contrast when wetted with wound exudate, enabling the nurse staff to immediately recognize when the absorption capacity of the wound dressing has been reached. Surprisingly it has been found that a green color similar to RAL6019 provides the strongest color contrast with wound exudate while simultaneously signaling cleanness and purity.

The first and the second layer of the envelope of the absorbent pad are joined to each other. This joining can be formed by any suitable process for the joining of materials, directly or indirectly.

The first and the second layer of the envelope each comprise a material exhibiting thermoplastic properties. The first and the second layer of the envelope are joined by a thermic process. It is possible to join the first layer and the second layer by a thermic process along a joining line. Thermic processes for the joining of materials can be performed in a continuous manner and therefore can be more cost-effective than other procedures. Suitable thermic processes for the joining of materials include heat welding, laser welding and ultrasonic welding.

The second layer of the envelope comprises a thermoplastic material that has hydrophobic properties and the first layer of the envelope comprises a thermoplastic material that is the same material as in the first layer and that has been treated chemically and/or physically to exhibit hydrophilic properties. If the first and the second layer comprises materials of different chemical nature, the physical properties of these materials can differ significantly. This can be disadvantageous in a joining process where conditions must be found that are compatible for each of the materials. In a thermic joining process the temperature must be higher than the melting points of each material. However, thermally sensitive materials can degrade during this procedure if the process temperature is too high. Therefore, depending on the chemical nature of the materials of the first and the second layer the choice of manufacturing processes may be severely restricted. If the first and the second layer comprise a material that has the same chemical nature, the materials do not differ in their physical properties relevant for a connection procedure. This is especially advantageous in a thermic joining process, when the process temperature only has to be slightly above the softening temperature of the material, and the risk for thermally degradation of the material is significantly reduced.

In a preferred embodiment the thermoplastic material of both the first layer and the second layer comprises polypropylene.

The first layer and the second layer of the envelope surrounding the envelope have been joined by a thermic process and therefore comprise a welding connection. A welding connection resulting from a thermic joining process exhibits a favorable connection characterized by its welding strength and its flexibility. Welding strength within the meaning of this application is the force that is necessary to separate two layers that are joined with each other. Welding strength can be determined by a method described below in the example part of this application. In a preferred embodiment the welding strength is higher than 0.75N/ 15mm, preferably higher than 1N/ 15mm.

A high welding strength is advantageous in order to prevent undesired delamination of the joined materials. Delamination of the first and the second layer of the absorbent core even in a minor extent can cause leakage. In that case wound exudate that has already been absorbed within the absorbent core could diffuse in outer zones of the wound dressing. It could contact intact skin surrounding the wound via a passage through the perforations in the wound contacting layer or it could leak from the wound dressing. This can cause maceration of intact skin surrounding the wound area. Furthermore, a leakage of wound exudate would cause a weakening of the adhesive connection between the backing layer and the perforated wound contact layer. If the leaked wound exudate diffuses along the distal side of the absorbent core that can cause the formation of wrinkles in the backing material which causes a discomfortable feeling for the user.

Flexibility within the meaning of this application is the property that enables a material to bend and to change its conformation. It is characterized by the stiffness.

Flexibility is important for the dressing to be able to adapt to an irregular body surface. Furthermore, a non-flexible dressing is very stiff and causes pressure on the patient's body surface during movement resulting in discomfort or pain.

The welding connection comprises at least one non-continuous welding line. A non-continuous welding line exhibits a flexibility which is favorable for the patient's comfort.

In a preferred embodiment, the welding connection comprises four to six parallel non-continuous welding lines resulting in a favorably balanced relationship of both properties, welding strength and flexibility.

In a preferred embodiment the at least one welding line is arranged parallel to the machine direction of the manufacturing process.

The wound contact layer comprises a layer of skin-friendly silicone adhesive. Suitably, the silicone composition is a so-called soft skin adhesive silicone elastomer. The total coating weight of the silicone is suitably from about 15 g/ m² to about 500 g/ m², preferably 50 g/ m² to 250 g/ m², more preferably 100g/ m² to 200 g/ m².

Surprisingly it has been found that a wound dressing according to claim 1 exhibits advantageous properties in the adhesive peel tests on skin described below in the example part of this application. A proper wound dressing must adhere to the skin without losing adhesiveness, which allows a use of the dressing for several hours, preferably for at least one day, more preferably for at least three days, most preferably for up to seven days. On the other hand, the dressing must exhibit skin-friendly properties that allow the dressing to be removed without causing pain to the patient or irritation to the wound or the surrounding skin. Surprisingly, it has been found that a dressing exhibiting a peel value on skin of between 200 mN/ cm, preferably 350 mN/ cm and 650 mN/ cm is advantageous in this manner.

Although it is possible to provide a wound contact layer consisting essentially of a soft skin adhesive silicone elastomer, the wound contact layer preferably comprises a further layer of a perforated sheet material wherein the perforations of the sheet material correspond to the apertures of the silicone adhesive without being occluded by the silicone adhesive. The perforated sheet material may be any medically acceptable perforated sheet material, including textile materials. Suitably, the perforated sheet material is a unitary sheet material such as a polymer mesh or an apertured polymer film. Suitable polymer materials include polyethylene, polypropylene, polyester, polyvinyl acetate, ethylene vinyl acetate and polyurethane. Suitably, the sheet material has a thickness of 1 µm to 100 mm, preferably 5 µm to 50 µm. A preferred sheet material is a polyurethane material available under the tradename Acrysil^{®} covered with 150 g/ m² silicone adhesive from the company Zodiac (France).

The wound contact layer comprises apertures for the passage of wound exudate to the absorbent core. Apertures can have any geometrical shape, regular or irregular, in particular a circular, oval, triangular, square, rectangular, pentangular, hexagonal or other polygonal shape with uniform or non-uniform side length.

In a preferred embodiment the wound contact layer has an open area of between 10 % to 25% of the entire area of the wound contact layer. Surprisingly it has been found that an open area in this range results in a favorable absorption velocity of wound exudate. Absorption velocity within the meaning of this application means the time of the absorption of wound exudate. It can be determined by a test method described in the example part of this application.

In a preferred embodiment the wound contact layer comprises apertures of any of these shapes having an average open area of from 0,03 mm² to 7,0 mm².

In a preferred embodiment the wound contact layer comprises apertures having an essentially circular shape and having an average diameter of between 0,2 mm to 3,0 mm. Surprisingly it has been found that the absorption velocity is favorable for a wound contact layer comprising apertures having a circular shape and an average diameter of 2,2 mm to 2,8 mm.

Suitably, the open area of the aperture layer is from about 10 % to 25 %. Surprisingly it has been found that the absorption velocity is favorable for a wound contact layer having an open area of 11 % to 22 %, preferably 12 % to 18 %. Suitably, the density of the apertures is from about 1000 to 100000 apertures per m², for example about 5000 to 50000 apertures per m².

Surprisingly it has been found that a wound dressing according to claim 1 exhibits advantageous properties in the absorption velocity test which is described below in the example part of this application.

Surprisingly, it has been found that a wound dressing according to claim 1 exhibits advantageous properties in respect to the balance between adhesiveness to skin and absorption velocity.

The backing layer, the absorbent pad and the wound contact layer can have different or similar extensions.

In one embodiment the dressing comprises a backing layer and an absorbent pad having a proximal and a distal surface. The distal surface of the absorbent pad is covered by the backing layer. The backing layer has an extension in both area directions that is larger than the area of the distal surface of the absorbent pad, while the absorbent pad and the wound contact layer are co-extensive. The backing layer therefore forms a border area completely surrounding the absorbent pad. The surrounding border area can be covered with a skin-friendly adhesive in order to build a dressing having a central absorbent pad and an adhesive border. This type of dressing is called "island-type" dressing.

In another embodiment the dressing comprises a backing layer and an absorbent pad having a proximal and a distal surface. The distal surface of the absorbent pad is covered by the backing layer. The backing layer has an extension in both area directions that is larger than the area of the distal surface of the absorbent pad. The backing layer therefore forms a border area completely surrounding the absorbent pad. The wound contact layer has an extension in both area directions that is larger than the area of the proximal surface of the absorbent pad, thereby forming a border area completely surrounding the absorbent pad, while the wound contact layer is coextensive with the backing layer. This type of dressing is called "sandwich-type" dressing. The latter type of dressing has a lower risk for delamination.

The absorbent pad can be covered by a further wound contact layer on its proximal surface.

In a preferred embodiment the absorbent core exhibits an absorption capacity of at least 100 g/ 100 cm², preferably more than 120 g/ 100 cm², more preferably more than 130 g/ 100 cm² according to the test method described within the example part of this application.

In a preferred embodiment the absorbent core comprises a mixture of absorbent fibers and superabsorbent materials. Absorbent fibers cause a quick absorption of wound exudate while superabsorbent materials exhibit a high absorption capacity. Suitable absorbent fibers can be cellulose-derived materials. In a preferred embodiment the absorbent fibers consist essentially of cellulose fibers. Suitable superabsorbent materials can be in the form of particles, gels or fibers. If the superabsorbent material is in the form of fibers, the fibers can be loosely distributed within the absorbent fibers or can be in a textile material. The textile material can comprise solely superabsorbent fibers or can further comprise absorbent fibers. The textile material can be in the form of any textile material that is suitable for incorporation into a wound dressing, e.g. nonwovens, woven, warp-knitted or weft-knitted materials.

In a preferred embodiment the absorbent core comprises a mixture of absorbent fibers and superabsorbent particles.

The wound dressing, the backing layer, the absorbent core, the absorbent pad, the envelope and the wound contact layer each can have any suitable size and basic geometrical shape, particularly squares, rectangles, circles, ovals, polygons. If the basic geometrical shape of any of these layers comprises corners, these corners preferably are rounded. This reduces the risk of curling of the layer and of delamination from other layers or skin. In a preferred embodiment, the radius for the rounded corners is from 5 mm to 15 mm, more preferably 12,5 mm.

In a preferred embodiment the first layer of the envelope and the second layer of the envelope are joined along the border area surrounding the absorbent core in a joining area, the joining area having an inner margin and an outer margin, and that the envelope comprises a space between the first and the second layer of the envelope, wherein the absorbent core is placed, the space being limited by the inner margin of the joining area, and the space having an area A₁, the absorbent core having an area A₂, wherein the area A₂ is at least 80 % of the area A₁.

The dressing according to the present invention may further comprise at least one removable cover sheet to cover the adhesive areas on the proximal surface of the wound dressing. The cover sheet covers and protects the absorbent pad and prevents premature adhesion of the adhesive parts of the wound dressing. It may comprise a film of polyethylene, polypropylene or fluorocarbons and paper coated with these materials or silicone. A suitable material for a cover sheet can be a polyethylene foil having a thickness of 100 µm and can be purchased from the company Flextrus^{®}.

Figure 1 shows a wound dressing in a top view of a preferred embodiment of the invention. Figure 2 shows the wound dressing of figure 1 in a cross-section view along the cutting line A-A of figure 1.

Figure 3 shows a wound dressing in a cross-section view representing an island-type embodiment of the invention.

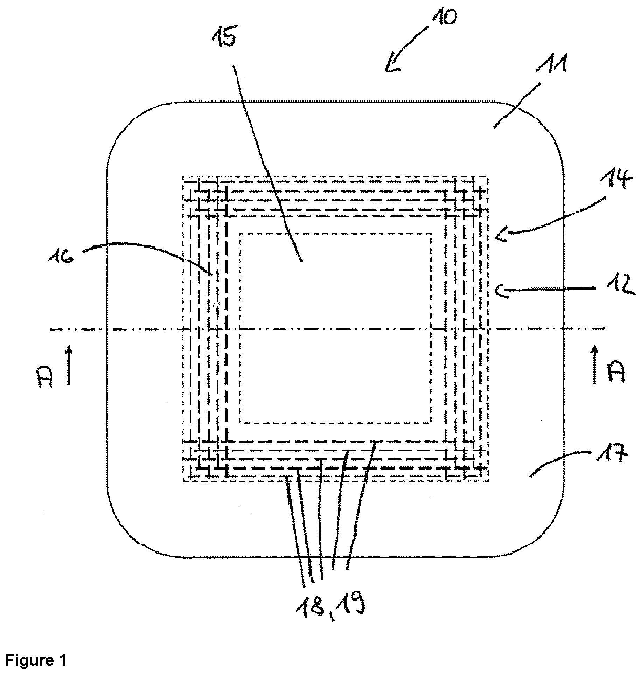

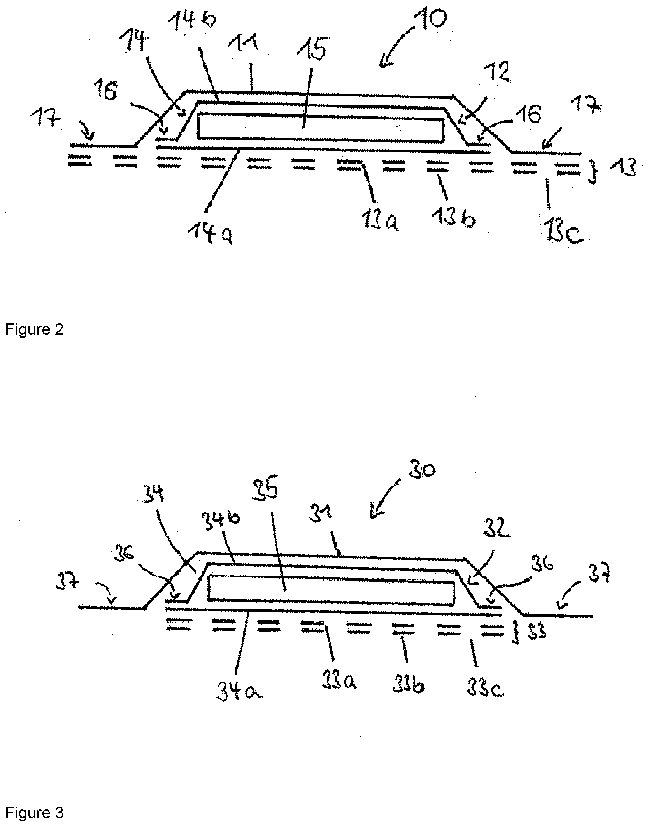

Figure 1 shows a top view of a wound dressing (10) having a backing layer (11) and an absorbent pad (12). A wound contact layer is present on the side of the dressing facing away from the viewer. The absorbent pad (12) comprises an absorbent core (15) and an envelope (14) surrounding the absorbent core (15). The envelope (14) comprises a first, proximal layer (facing away from the viewer) of a liquid-permeable nonwoven material and a second, distal layer (14b, see figure 2) of a substantially liquid-impermeable nonwoven material. The proximal layer of the envelope (14) covers the proximal side of the absorbent core (15) and extends over the proximal side of the absorbent core (15), thereby forming a border area (16) surrounding the absorbent core (15). The distal layer (14b) of the envelope (14) covers the distal side of the absorbent core (15) and extends over the distal side of the absorbent core (15), thereby forming a border area (16) surrounding the absorbent core (15). The proximal layer of the envelope (14) and the distal layer (14b) of the envelope (14) are joined along the border area (16) surrounding the absorbent core (15) by a thermic process and therefore comprise a welding connection (18). The welding connection (18) is formed by five non-continuous welding lines (19). The backing layer (11) extends over the absorbent pad (12), thereby forming a border area (17) surrounding the absorbent pad (12).

Figure 2 shows a cross-sectional view of the wound dressing (10) of figure 1 along the cutting line A-A from figure 1, having an absorbent pad (12), a backing layer (11) and a wound contact layer (13). The absorbent pad (12) comprises an absorbent core (15) and an envelope (14). The envelope (14) is formed from a proximal layer (14a) and a distal layer (14b). The wound contact layer (13) is formed from a layer of a perforated sheet material (13a) and a layer of skin-friendly silicone adhesive (13b). The backing layer (11) is made from a vapour-permeable and liquid-impermeable polyurethane film material with low friction characteristics having a thickness of 30 µm. The backing layer (11) is coated with a layer of acrylate-based adhesive (not shown). The absorbent core (15) comprises a mixture of cellulose fibers and superabsorbent sodium polyacrylate particles in a pre-fabricated airlaid material. The absorbent core (15) further comprises a diffusion layer (not shown) in form of a sheet of cellulose tissue that is wrapped around the airlaid mixture of cellulose fibers and polyacrylate particles. The proximal layer (14a) of the envelope (14) is a nonwoven made from a mixture of viscose fibers and polyamide fibers. The distal layer (14b) of the envelope (14) is a nonwoven made from polypropylene fibers. The proximal layer (14a) of the envelope (14) covers the proximal side of the absorbent core (15) and extends over the proximal side of the absorbent core (15), thereby forming a border area (16) surrounding the absorbent core (15). The distal layer (14b) of the envelope (14) covers the distal side of the absorbent core (15) and extends over the distal side of the absorbent core (15), thereby forming a border area (16) surrounding the absorbent core (15). The proximal layer (14a) of the envelope (14) and the distal layer (14b) of the envelope (14) are joined along the border area (16) surrounding the absorbent core (12). The perforated sheet material (13a) of the wound contact layer (13) is made from a polyurethane film that comprises apertures (13c) having a circular shape. These apertures (13c) have similar shapes having an average diameter of 2,4mm and are arranged in a regular pattern resulting in an open area of 15 %. The backing layer (11) extends over the absorbent pad (12), thereby forming a border area (17) surrounding the absorbent pad (12). The wound contact layer (13) extends over the absorbent pad (12), thereby forming a border area (17) surrounding the absorbent pad (12). The backing layer (11) and the wound contact layer (13) are coextensive.

Figure 3 shows a cross-sectional view of a wound dressing (30) of an island-type having an absorbent pad (32), a backing layer (31) and a wound contact layer (33). The absorbent pad (32) comprises an absorbent core (35) and an envelope (34). The envelope is formed from a proximal layer (34a) and a distal layer (34b). The wound contact layer (33) is formed from a layer of a perforated sheet material (33a) and a layer of skin-friendly silicone adhesive (33b). The backing layer (31) is made from a vapour-permeable and liquid-impermeable polyurethane film material with low friction characteristics having a thickness of 30 µm. The backing layer (31) is coated with a layer of acrylate-based adhesive (not shown). The absorbent core (35) comprises a mixture of cellulose fibers and superabsorbent sodium polyacrylate particles in a pre-fabricated airlaid material. The absorbent core (35) further comprises a diffusion layer (not shown) in form of a sheet of cellulose tissue that is wrapped around the airlaid mixture of cellulose fibers and polyacrylate particles. The proximal layer (34a) of the envelope (34) is a nonwoven made from a mixture of viscose fibers and polyamide fibers. The distal layer (34b) of the envelope (34) is a nonwoven made from polypropylene fibers. The proximal layer (34a) of the envelope (34) covers the proximal side of the absorbent core (35) and extends over the proximal side of the absorbent core (35), thereby forming a border area (36) surrounding the absorbent core (35). The distal layer (34b) of the envelope (34) covers the distal side of the absorbent core (35) and extends over the distal side of the absorbent core (35), thereby forming a border area (36) surrounding the absorbent core (35). The proximal layer (34a) of the envelope (34) and the distal layer (34b) of the envelope (34) are joined along the border area (36) surrounding the absorbent core (35). The perforated sheet material (33a) of the wound contact layer (33) is made from a polyurethane film that comprises apertures (33c) having a circular shape. These apertures (33c) have similar shapes having an average diameter of 2,4 mm and are arranged in a regular pattern resulting in an open area of 15 %. The wound contact layer further comprises a layer of skin-friendly silicone adhesive (33b). The backing layer (31) extends over the absorbent pad (32), thereby forming a border area (37) surrounding the absorbent pad (32).

### Examples

### Example 1: Wound dressing

The wound dressing has an absorbent pad, a backing layer and a wound contact layer. The absorbent pad comprises an absorbent core and an envelope. The envelope is formed from a proximal layer and a distal layer. The wound contact layer is formed from a layer of a perforated sheet material and a layer of skin-friendly silicone adhesive. The backing layer is made from a vapour-permeable and liquid-impermeable polyurethane film material with low friction characteristics having a thickness of 30 µm. The absorbent core comprises a mixture of cellulose fibers and superabsorbent sodium polyacrylate particles in a pre-fabricated airlaid material. The absorbent core further comprises a diffusion layer in form of a sheet of cellulose tissue that is wrapped around the airlaid mixture of cellulose and polyacrylate particles. The proximal layer of the envelope is a nonwoven made from a mixture of viscose and polyamide fibers. The distal layer of the envelope is a nonwoven made from polypropylene fibers. The perforated sheet material of the wound contact layer is made from a polyurethane film that comprises apertures having a circular shape. These apertures exhibit similar shapes having an average diameter of 2,4 mm and are arranged in a regular pattern resulting in an open area of 15 %. The wound contact layer further comprises a layer of skin-friendly silicone adhesive without occluding the apertures of the perforated sheet material.

### Example 2: Test solutions used in the characterization of wound dressings

### Solution A (saline solution)

2L Deionized water
0,74 g Calcium chloride dihydrate (CaCl₂ • 2 H₂O, CAS: 10035-04-5)
16,6 g Sodium chloride (NaCl, CAS: 7647-14-5)

### Solution B (exudates solution)

1 L Deionized water
70 g Albumin from chicken egg white (N° CAS 9006-59-1)
0,2 g Allura Red AC (CAS 25956-17-6)
9 g Sodium chloride (NaCl, N° CAS: 7647-14-5)
0,37 g Calcium chloride dihydrate (CaCl₂ • 2 H₂O, N° CAS: 10035-04-5)
2 g Methyl 4-hydroxybenzoate (CAS: 99-76-3)
1 g Propyl 4-hydroxybenzoate (CAS: 94-13-3)

### Example 3: Test method absorption velocity

The absorption velocity of a dressing is determined by the time necessary to completely absorb a test solution. Test solutions can be either saline solution (solution A) or exudate solution (solution B). Solutions (saline solution or exudates solution) as well as test products must be preconditioned at room temperature prior to testing by leaving the test products and the solutions for two hours at 22 °C.

A 50 mL buret is filled with the test solution. The liquid level should be fixed at 15mL.

The dressing is placed under the buret, that surface which in use faces the wound now faces the buret. The distance between the buret and the dressing is adjusted to 1 cm. The tap of the buret is opened, and simultaneously a stopwatch is started, while 2 mL of test solution are allowed to flow out. The stopwatch is stopped immediately when the 2 mL of test solution are completely absorbed by the dressing, i.e. when no drop is remaining over the perforation of the wound contact layer. If the dressing is big enough, 1 to 3 measurements can be made on the same dressing.

Positions of the tests on a dressing are one in the centre, two further points along a diagonal in direction to the corners of the dressing, similar to the position of the three points on a regular die.

At least five samples have to be investigated.

Each value has to be classified in the following categories

| Time (s) | [0 to 4,9] | [5,0 to10,9] | [11,0 to 30,9] | [31,0 to 60,0] | >60 |
|---|---|---|---|---|---|
| category | Immediate | Very quick | quick | Average | slow |

A dressing according to the invention has been compared with commercially available competitor dressings Biatain^{®} silicone, Allevyn^{®} Life, Mepilex^{®} border (which is considered as being a dressing according to the disclosure of WO93/19709 and WO93/19710).

Absorption velocity:

| **Dressing** | **Absorption velocity [percentage of samples]** | | | | |
|---|---|---|---|---|---|
| | immediate | Very quick | quick | average | slow |
| Example 1 | 20 % | 80 % | 0% | 0% | 0% |
| Biatain^{®} silicone | 0% | 0% | 0% | 20% | 80 % |
| Allevyn^{®} Life | 0% | 0% | 100 % | 0% | 0% |
| Mepilex^{®} border | 0% | 0% | 0% | 100 % | 0% |

### Example 4: Test method absorption capacity

Solutions (saline solution or exudates solution) as well as test products must be preconditioned at room temperature prior to testing by leaving the test products and the test solutions for two hours at 22 °C.

The basic mass (m₁) of a dressing is determined after removal of the release liners.

The length and width of the absorbent core is determined so that the absorbent core surface (S) can be determined. A bowl is filled with the test solution. The weight of testing solution should be at least 40 times higher than the dressing itself. The dressing is put into the bowl, and simultaneously a stopwatch is started. That side of the dressing which in use faces the wound side should face the bottom of the bowl, the back side of the dressing should be on the top. The dressing should not be glued to the bottom of the bowl. The dressing is left within the bowl for 30 min +/- 1 min. After 30 min, the dressing is removed from the bowl. The dressings should only be manipulated via the borders and not the absorbent core itself. The dressings are fixed on one corner to a stand with a clamp and allowed to hang for 20 min at room temperature. The wet mass (m₂) of the dressing is determined. The amount of liquid (M_{liquid}) absorbed is calculated: ${m}_{liquid} = {m}_{2} - {m}_{1}$

The absorption capacity is the amount of liquid absorbed (m₂-m₁) by the absorbent core surface (S) and is given in g/ 100 cm²: $absorption capacity = {m}_{2} - {m}_{1} / S \times 100$

A dressing according to the invention has been compared with commercially available competitor dressings Biatain^{®} silicone, Allevyn^{®} Life, Mepilex^{®} border (which is considered as being a dressing according to the disclosure of WO93/19709 and WO93/19710).

Comparison of absorption capacities:

| **Dressing** | **Absorption capacity in g/ 100 cm²** |
|---|---|
| Example 1 | 147 |
| Biatain^{®} silicone | 116 |
| Allevyn^{®} Life | 87 |
| Mepilex^{®} border | 54 |

### Example 5: Test method for adhesiveness on skin - Peel test

Subjects came to the Study Site. They were informed about the study and gave their written consent. Test products were applied to the back of each subject (two samples per test product): One sample of each test product was adhered to the upper part of the back. The second sample of each test product was adhered to the lower part of the back at the same position as the first sample in the upper part.

The lower end of each sample was folded on the length of approximately 0.5 cm, in order to obtain a starting point for removing the sample from downside to upside with the universal test machine. The test materials were applied by a technician and pressed to the skin with the use of a metal roll (1 kg, 5 times of rolling back and forth).

Subjects came to the Study Site 3 hours and 50 minutes after the product application. They stayed in the air-conditioned room for at least 10 minutes. It was checked that the subjects did not sweat. Then, samples were removed 4 hours after application by using a universal test machine, set up for determining adhesive forces.

Universal Test Machine: Zwick 1120 (Zwick GmbH, Ulm, Germany). The universal test machine measures the force needed to pull off the adhesive-coated backings from the skin. For removal of the samples the subjects were positioned in a sitting position. The samples were removed under an angle of approximately 135 °.

1 measurement per sample, 2 samples per product

### Example 6: Test method for welding strength

The test for welding strength is carried out with Tensile Tester MTS C42.503E (MTS Systems Corporation, Eden Prairie, USA); cell strength 50N.

Test samples are prepared by stamping a part of the absorbent pad including the welding line having a rectangular shape of 15 mm in width and 25 mm in length, wherein the 15 mm side corresponds to the welding line. The sample must be at a distance of at least 2 mm from the corner. After stamping, the absorbent material is removed. The sample to be tested is only made of 2 nonwovens welded together. The clamp jaws of the tensile tester are placed in a way that the distance between the 2 jaws is 2 cm. Each nonwoven is placed in a separate jaw. The tensile tester is started at a speed of 200 mm/min until the 2 nonwovens are separated. This can happen either after a complete break of the weld or after complete tearing of the nonwovens themselves.

The welding strength is the average strength over the testing period. It is given in N/ 15mm.

## Claims

1. Wound dressing (10, 30) comprising a backing layer (11, 31), an absorbent pad (12, 32) and a wound contact layer (13, 33), wherein the backing layer (11, 31) comprises a water vapour-permeable and substantially liquid-impermeable film material, wherein the absorbent pad (12, 32) comprises an absorbent core (15, 35) having a proximal side and a distal side, and an envelope (14, 34) surrounding the absorbent core (15, 35), the absorbent core (15, 35) comprising an absorbent material, and wherein the wound contact layer (13, 33) comprises a layer of a skin-friendly silicone adhesive (13b, 33b), **characterized in that** the envelope (14, 34) comprises a first layer (14a, 34a) of a first liquid-permeable material and a second layer (14b, 34b) of a second material different from the first material, wherein the first layer (14a, 34a) of the envelope (14, 34) covers the proximal side of the absorbent core (15, 35) and wherein the second layer (14b, 34b) of the envelope (14, 34) covers the distal side of the absorbent core (15, 35), and wherein the first layer (14a, 34a) of the envelope (14, 34) extends over the proximal side of the absorbent core (15, 35) and the second layer (14b, 34b) of the envelope (14, 34) extends over the distal side of the absorbent core (15, 35) each of them forming a border area (16, 36) surrounding the absorbent core (15, 35), the first (14a, 34a) and second layer (14b, 34b) of the envelope (14, 34) being joined to each other along the border area (16, 36) surrounding the absorbent core (15, 35) wherein the second layer (14b, 34b) of the envelope (14, 34) comprises a thermoplastic material that has hydrophobic properties, and that the first layer (14a, 34a) of the envelope (14, 34) comprises a thermoplastic material that is the same material as in the second layer (14b, 34b) and that has been treated chemically and/or physically to exhibit hydrophilic properties, wherein the first (14a, 34a) and the second layer (14b, 34b) of the envelope (14, 34) comprise a material exhibiting thermoplastic properties and **in that** the first layer (14a, 34a) and the second layer (14b, 34b) of the envelope (14, 34) have been joined by a thermic process and therefore comprise a welding connection (18), **characterized in that** the welding connection (18) comprises at least one non-continuous welding line (19).

2. Wound dressing (10, 30) according to claim 1, **characterized in that** the second layer (14b, 34b) of the envelope (14, 34) comprises a substantially liquid-impermeable material.

3. Wound dressing (10, 30) according to at least one of the preceding claims, **characterized in that** the thermoplastic material of both the first layer (14a, 34a) and the second layer (14b, 34b) of the envelope (14, 34) comprises polypropylene.

4. Wound dressing (10, 30) according to at least one of the preceding claims, **characterized in that** the welding connection (18) comprises four to six parallel non-continuous welding lines (19).

5. Wound dressing (10, 30) according to claim 4, **characterized in that** the at least one welding line (19) is arranged parallelly to the machine direction of the manufacturing process.

6. Wound dressing (10, 30) according to at least one of the preceding claims, **characterized in that** the wound contact layer (13, 33) has an open area of between 10 to 25 % of the entire area of the wound contact layer (13, 33).

7. Wound dressing (10, 30) according to at least one of the preceding claims, **characterized in that** the wound contact layer (13, 33) comprises apertures (13c, 33c) having an essentially circular shape with an average diameter of between 0,2 mm to 3,0 mm.

8. Wound dressing (10, 30) according to at least one of the preceding claims, **characterized in that** the absorbent pad (14, 34) comprises a proximal surface and a distal surface and the backing layer (11, 31) comprises a coating of a pressure-sensitive adhesive, and that the backing layer (11, 31) extends over the distal surface of the absorbent pad (12, 32) forming an adhesive border area (17, 37) that surrounds the absorbent pad (12, 32).

9. Wound dressing (10) according to claim 8, **characterized in that** the wound contact layer (13) is coextensive with the backing layer (11).

10. Wound dressing (10, 30) according to at least one of the preceding claims, **characterized in that** the absorbent core (15, 35) comprises an absorption capacity of at least 100 g/ 100 cm² according to the method described in example 4.

11. Wound dressing (10, 30) according to at least one of the preceding claims, **characterized in that** the absorbent core (15, 35) comprises a mixture of absorbent fibers and superabsorbent particles.

12. Wound dressing (10, 30) according to at least one of the preceding claims, **characterized in that** the first layer (14a, 34a) of the envelope (14, 34) and the second layer (14b, 34b) of the envelope (14, 34) are joined along the border area (16, 36) surrounding the absorbent core (15, 35) in a joining area, the joining area having an inner margin and an outer end, and that the envelope (14, 34) comprises a space between the first (14a, 34a) and the second layer (14b, 34b) of the envelope (14, 34), wherein the absorbent core (15, 35) is placed, the space being limited by the inner margin of the joining area, and the space having an area A1, the absorbent core (15, 35) having an area A2, wherein the area A2 is at least 80 % of the area A1.

## Patentansprüche

1. Wundverband (10, 30) umfassend eine Trägerschicht (11, 31), ein absorbierendes Kissen (12, 32) und eine Wundkontaktschicht (13, 33), wobei die Trägerschicht (11, 31) ein wasserdampfdurchlässiges und im Wesentlichen flüssigkeitsundurchlässiges Folienmaterial umfasst, wobei das absorbierende Kissen (12, 32) einen absorbierenden Kern (15, 35) mit einer proximalen Seite und einer distalen Seite und eine absorbierende Hülle (14, 34), die den absorbierenden Kern (15, 35) umgibt, umfasst, wobei der absorbierende Kern (15, 35) ein absorbierendes Material umfasst, und wobei die Wundkontaktschicht (13, 33) eine Schicht aus einem hautfreundlichen Silikonklebstoff (13b, 33b) umfasst, **dadurch gekennzeichnet, dass** die Hülle (14, 34) eine erste Schicht (14a, 34a) aus einem ersten flüssigkeitsdurchlässigen Material und eine zweite Schicht (14b, 34b) aus einem zweiten Material, das sich von dem ersten Material unterscheidet, umfasst, wobei die erste Schicht (14a, 34a) der Hülle (14, 34) die proximale Seite des absorbierenden Kerns (15, 35) bedeckt und wobei die zweite Schicht (14b, 34b) der Hülle (14, 34) die distale Seite des absorbierenden Kerns (15, 35) bedeckt, und wobei sich die erste Schicht (14a, 34a) der Hülle (14, 34) über die proximale Seite des absorbierenden Kerns (15, 35) erstreckt und sich die zweite Schicht (14b, 34b) der Hülle (14, 34) über die distale Seite des absorbierenden Kerns (15, 35) erstreckt, wobei jede von ihnen einen Randbereich (16, 36) bildet, der den absorbierenden Kern (15, 35) umgibt, wobei die erste Schicht (14a, 34a) und die zweite Schicht (14b, 34b) der Hülle (14, 34) entlang des Randbereichs (16, 36), der den absorbierenden Kern (15, 35) umgibt, miteinander verbunden sind, wobei die zweite Schicht (14b, 34b) der Hülle (14, 34) ein thermoplastisches Material umfasst, das hydrophobe Eigenschaften aufweist, und dass die erste Schicht (14a, 34a) der Hülle (14, 34) ein thermoplastisches Material umfasst, das das gleiche Material wie in der zweiten Schicht (14b, 34b) ist und das chemisch und/oder physikalisch behandelt worden ist, um hydrophile Eigenschaften aufzuweisen, wobei die erste (14a, 34a) und die zweite Schicht (14b, 34b) der Hülle (14, 34) ein Material mit thermoplastischen Eigenschaften umfassen, und dass die erste Schicht (14a, 34a) und die zweite Schicht (14b, 34b) der Hülle (14, 34) durch ein thermisches Verfahren verbunden worden sind und daher eine Schweißverbindung (18) umfassen, **dadurch gekennzeichnet, dass** die Schweißverbindung (18) wenigstens eine nicht-durchgehende Schweißnaht (19) umfasst.

2. Wundverband (10, 30) nach Anspruch 1, **dadurch gekennzeichnet, dass** die zweite Schicht (14b, 34b) der Hülle (14, 34) ein im Wesentlichen flüssigkeitsundurchlässiges Material umfasst.

3. Wundverband (10, 30) nach wenigstens einem der vorstehenden Ansprüche, **dadurch gekennzeichnet, dass** das thermoplastische Material sowohl der ersten Schicht (14a, 34a) als auch der zweiten Schicht (14b, 34b) der Hülle (14, 34) Polypropylen umfasst.

4. Wundverband (10, 30) nach wenigstens einem der vorstehenden Ansprüche, **dadurch gekennzeichnet, dass** die Schweißverbindung (18) vier bis sechs parallele nicht-durchgehende Schweißnähte (19) umfasst.

5. Wundverband (10, 30) nach Anspruch 4, **dadurch gekennzeichnet, dass** die wenigstens eine Schweißnaht (19) parallel zu der Maschinenrichtung des Herstellungsverfahrens angeordnet ist.

6. Wundverband (10, 30) nach wenigstens einem der vorstehenden Ansprüche, **dadurch gekennzeichnet, dass** die Wundkontaktschicht (13, 33) eine offene Fläche zwischen 10 bis 25 % der gesamten Fläche der Wundkontaktschicht (13, 33) aufweist.

7. Wundverband (10, 30) nach wenigstens einem der vorstehenden Ansprüche, **dadurch gekennzeichnet, dass** die Wundkontaktschicht (13, 33) Öffnungen (13c, 33c) mit einer im Wesentlichen kreisförmigen Form mit einem mittleren Durchmesser zwischen 0,2 mm und 3,0 mm aufweist.

8. Wundverband (10, 30) nach wenigstens einem der vorstehenden Ansprüche, **dadurch gekennzeichnet, dass** das absorbierende Kissen (14, 34) eine proximale Oberfläche und eine distale Oberfläche umfasst und die Trägerschicht (11, 31) eine Beschichtung aus einem Haftklebstoff umfasst, und dass sich die Trägerschicht (11, 31) über die distale Oberfläche des absorbierende Kissens (12, 32) erstreckt und einen Kleberandbereich (17, 37) bildet, der das absorbierende Kissen (12, 32) umgibt.

9. Wundverband (10) nach Anspruch 8, **dadurch gekennzeichnet, dass** die Wundkontaktschicht (13) koextensiv mit der Trägerschicht (11) ist.

10. Wundverband (10, 30) nach wenigstens einem der vorstehenden Ansprüche, **dadurch gekennzeichnet, dass** der absorbierende Kern (15, 35) ein Absorptionsvermögen von wenigstens 100 g/100 cm² nach dem in Beispiel 4 beschriebenen Verfahren aufweist.

11. Wundverband (10, 30) nach wenigstens einem der vorstehenden Ansprüche, **dadurch gekennzeichnet, dass** der absorbierende Kern (15, 35) ein Gemisch aus absorbiereden Fasern und superabsorbierenden Partikeln umfasst.

12. Wundverband (10, 30) nach wenigstens einem der vorstehenden Ansprüche, **dadurch gekennzeichnet, dass** die erste Schicht (14a, 34a) der Hülle (14, 34) und die zweite Schicht (14b, 34b) der Hülle (14, 34) entlang des den absorbierenden Kern (15, 35) umgebenden Randbereichs (16, 36) in einem Verbindungsbereich verbunden sind, wobei der Verbindungsbereich einen Innenrand und ein Außenende aufweist, und dass die Hülle (14, 34) einen Raum zwischen der ersten (14a, 34a) und der zweiten Schicht (14b, 34b) der Hülle (14, 34) umfasst, worin der absorbierende Kern (15, 35) angeordnet ist, wobei der Raum durch den inneren Rand des Verbindungsbereichs begrenzt ist, der Raum eine Fläche A1 aufweist und der absorbierende Kern (15, 35) eine Fläche A2 aufweist, wobei die Fläche A2 wenigstens 80 % der Fläche A1 beträgt.

## Revendications

1. Pansement (10, 30) comprenant une couche de doublure (11, 31), une compresse absorbante (12, 32) et une couche de contact avec la plaie (13, 33), dans lequel la couche de doublure (11, 31) comprend un matériau en film perméable à la vapeur d'eau et sensiblement imperméable aux liquides, dans lequel la compresse absorbante (12, 32) comprend un cœur absorbant (15, 35) ayant un côté proximal et un côté distal, et une enveloppe (14, 34) entourant le cœur absorbant (15, 35), le cœur absorbant (15, 35) comprenant un matériau absorbant, et dans lequel la couche de contact avec la plaie (13, 33) comprend une couche d'un adhésif à base de silicone respectueux de la peau (13b, 33b), **caractérisé en ce que** l'enveloppe (14, 34) comprend une première couche (14a, 34a) d'un premier matériau perméable aux liquides et une deuxième couche (14b, 34b) d'un deuxième matériau différent du premier matériau, dans lequel la première couche (14a, 34a) de l'enveloppe (14, 34) recouvre le côté proximal du cœur absorbant (15, 35) et dans lequel la deuxième couche (14b, 34b) de l'enveloppe (14, 34) recouvre le côté distal du cœur absorbant (15, 35), et dans lequel la première couche (14a, 34a) de l'enveloppe (14, 34) s'étend par-dessus le côté proximal du cœur absorbant (15, 35) et la deuxième couche (14b, 34b) de l'enveloppe (14, 34) s'étend par-dessus le côté distal du cœur absorbant (15, 35), chacune d'elles formant une zone de bordure (16, 36) entourant le cœur absorbant (15, 35), la première (14a, 34a) et la deuxième couche (14b, 34b) de l'enveloppe (14, 34) étant reliées l'une à l'autre le long de la zone de bordure (16, 36) entourant le cœur absorbant (15, 35), la deuxième couche (14b, 34b) de l'enveloppe (14, 34) comprenant un matériau thermoplastique qui possède des propriétés hydrophobes, et **en ce que** la première couche (14a, 34a) de l'enveloppe (14, 34) comprend un matériau thermoplastique qui est le même matériau que dans la deuxième couche (14b, 34b) et **en ce qu'**elle a été traitée chimiquement et/ou physiquement pour présenter des propriétés hydrophiles, dans lequel la première (14a, 34a) et la deuxième couche (14b, 34b) de l'enveloppe (14, 34) comprennent un matériau présentant des propriétés thermoplastiques et **en ce que** la première couche (14a, 34a) et la deuxième couche (14b, 34b) de l'enveloppe (14, 34) ont été reliées par un processus thermique et comprennent par conséquent un raccord soudé (18), **caractérisé en ce que** le raccord soudé (18) comprend au moins une ligne de soudure non continue (19).

2. Pansement (10, 30) selon la revendication 1, **caractérisé en ce que** la deuxième couche (14b, 34b) de l'enveloppe (14, 34) comprend un matériau sensiblement imperméable aux liquides.

3. Pansement (10, 30) selon au moins une des revendications précédentes, **caractérisé en ce que** le matériau thermoplastique à la fois de la première couche (14a, 34a) et de la deuxième couche (14b, 34b) de l'enveloppe (14, 34) comprend du polypropylène.

4. Pansement (10, 30) selon au moins une des revendications précédentes, **caractérisé en ce que** le raccord soudé (18) comprend quatre à six lignes de soudure non continues parallèles (19).

5. Pansement (10, 30) selon la revendication 4, **caractérisé en ce que** l'au moins une ligne de soudure (19) est disposée parallèlement au sens machine du procédé de fabrication.

6. Pansement (10, 30) selon au moins une des revendications précédentes, **caractérisé en ce que** la couche de contact avec la plaie (13, 33) a une zone ouverte représentant entre 10 et 25 % de la superficie entière de la couche de contact avec la plaie (13, 33).

7. Pansement (10, 30) selon au moins une des revendications précédentes, **caractérisé en ce que** la couche de contact avec la plaie (13, 33) comprend des ouvertures (13c, 33c) ayant une forme essentiellement circulaire avec un diamètre moyen compris entre 0,2 mm et 3,0 mm.

8. Pansement (10, 30) selon au moins une des revendications précédentes, **caractérisé en ce que** la compresse absorbante (14, 34) comprend une surface proximale et une surface distale et la couche de doublure (11, 31) comprend un revêtement d'un adhésif sensible à la pression, et **en ce que** la couche de doublure (11, 31) s'étend par-dessus la surface distale de la compresse absorbante (12, 32), formant une zone de bordure adhésive (17, 37) qui entoure la compresse absorbante (12, 32).

9. Pansement (10) selon la revendication 8, **caractérisé en ce que** la couche de contact avec la plaie (13) est coextensive avec la couche de doublure (11).

10. Pansement (10, 30) selon au moins une des revendications précédentes, **caractérisé en ce que** le cœur absorbant (15, 35) présente une capacité d'absorption d'au moins 100 g/100 cm² selon la méthode décrite dans l'exemple 4.

11. Pansement (10, 30) selon au moins une des revendications précédentes, **caractérisé en ce que** le cœur absorbant (15, 35) comprend un mélange de fibres absorbantes et de particules superabsorbantes.

12. Pansement (10, 30) selon au moins une des revendications précédentes, **caractérisé en ce que** la première couche (14a, 34a) de l'enveloppe (14, 34) et la deuxième couche (14b, 34b) de l'enveloppe (14, 34) sont reliées le long de la zone de bordure (16, 36) entourant le cœur absorbant (15, 35) dans une zone de liaison, la zone de liaison ayant une marge intérieure et une extrémité extérieure, et **en ce que** l'enveloppe (14, 34) comprend un espace entre la première (14a, 34a) et la deuxième couche (14b, 34b) de l'enveloppe (14, 34) dans lequel est placé le cœur absorbant (15, 35), l'espace étant limité par la marge intérieure de la zone de liaison, et l'espace ayant une superficie A1, le cœur absorbant (15, 35) ayant une superficie A2, dans lequel la superficie A2 représente au moins 80 % de la superficie A1.
